(19) Europäisches Patentamt
European Patent Office
Office européen des brevets

(11) Publication number: 0 648 734 A1

(12) # EUROPEAN PATENT APPLICATION

(21) Application number: 94307138.1

(51) Int. Cl.[6]: C07C 67/055, C07C 69/01

(22) Date of filing: 29.09.94

(30) Priority: 30.09.93 US 129780

(43) Date of publication of application:
19.04.95 Bulletin 95/16

(84) Designated Contracting States:
BE DE ES FR GB IT NL

(71) Applicant: UNION CARBIDE COATINGS SERVICE TECHNOLOGY CORP.
39 Old Ridgebury Road
Danbury, CT 06817-0001 (US)

(72) Inventor: Packett, Diana Lee
1020 Chestnut Street,
South Charleston
State of West Virginia, 25309 (US)

(74) Representative: Allard, Susan Joyce et al
BOULT, WADE & TENNANT
27 Furnival Street
London EC4A 1PQ (GB)

(54) **Synthesis of higher vinyl esters from ethylene and higher carboxylic acids.**

(57) A homogeneous liquid phase process for producing a vinyl carboxylate in which the carboxylate group contains at least four carbon atoms, which process comprises (1) forming a homogeneous liquid reaction mixture containing ethylene, oxygen carboxylic acid having at least three carbon atoms, a palladium (II) catalyst and a copper (II) oxidant and (2) maintaining the reaction mixture under conditions at which the ethylene and the carboxylic acid react to form the vinyl carboxylate in the homogeneous liquid reaction mixture, with the proviso that at least one of the following conditions exists during said process :

(i) the copper (II) oxidant is a copper (II) carboxylate in which carboxylate group has a molecular weight that is higher than the molecular weight of the carboxylate group in the carboxylic acid ;

(ii) the palladium (II) catalyst is formed in situ in the reaction mixture by the oxidation of a palladium (0) colloid ; and/or

(iii) the reaction mixture also contains a polyglyme compound as a promoter.



Jouve, 18, rue Saint-Denis, 75001 PARIS

## Field of The Invention

This invention relates to the production of higher vinyl esters (e.g., vinyl 2-ethylhexanoate) and, in particular, to a homogeneous liquid phase process for producing such esters from ethylene and higher carboxylic acids (e.g., 2-ethylhexanoic acid).

## Description of Related Art

Vinyl esters (e.g., vinyl acetate) are valuable starting materials for use in producing polymers. Higher vinyl esters (i.e., vinyl esters of carboxylic acids containing at least four carbon atoms) are more desirable than vinyl acetate in applications where it desired that polymers produced from the esters have glass transition temperatures different from the glass transition temperatures that can be obtained using vinyl acetate.

Vinyl acetate is conventionally produced by reacting ethylene and acetic acid in a gas phase reaction mixture. However, similar gas phase processes involving ethylene and higher carboxylic acids are not completely satisfactory because the higher boiling points of the higher carboxylic acids makes them more difficult to volatilize and because the resulting higher vinyl esters makes them more difficult to separate from the other components of the reaction mixture. Hence vinyl esters of higher carboxylic acids are usually produced in the liquid phase by transvinylation of the higher carboxylic acid with vinyl acetate or by reacting the higher carboxylic acid with acetylene. A drawback of the former (transvinylation) process is that two reactions (vinylation and transvinylation) are required to convert ethylene to the desired ester while a drawback of the latter (acid/acetylene) process is that acetylene is more expensive than ethylene.

Efforts have been made to overcome the above problems by providing homogeneous liquid phase processes for producing vinyl carboxylates in which the carboxylate groups contains at least four carbon atoms by: (1) forming a homogeneous liquid mixture containing ethylene, oxygen, a carboxylic acid having at least four carbon atoms, a palladium (II) catalyst and a copper (II) oxidant and (2) maintaining the mixture under conditions at which the ethylene and the carboxylic acid react to form the vinyl carboxylate in the homogeneous liquid mixture. However, such processes have not been entirely satisfactory in that catalyst activity and stability leave room for improvement and cocatalysts may be required.

It is an object of the present invention to provide an improved process for producing higher vinyl esters directly from ethylene and higher carboxylic acids.

Other objects of the present invention will be apparent from the description thereof appearing below.

## Summary of Invention

This invention provides a homogeneous liquid phase process for producing a vinyl carboxylate in which the carboxylate group contains at least four carbon atoms, which process comprises: (1) forming a homogeneous liquid reaction mixture containing ethylene, oxygen, a carboxylic acid having at least three carbon atoms, a palladium (II) catalyst and a copper (II) oxidant and (2) maintaining the reaction mixture under conditions at which the ethylene and the carboxylic acid react to form the vinyl carboxylate in the homogeneous liquid mixture, with the proviso that at least one of the following conditions exists during said process:

(i) the copper (II) oxidant is a copper (II) carboxylate in which carboxylate group has a molecular weight that is higher than the molecular weight of the carboxylate group in the carboxylic acid;

(ii) the palladium (II) catalyst is formed in situ in the reaction mixture by the oxidation of a palladium (0) colloid; and/or

(iii) the reaction mixture also contains a polyglyme compound as a promoter.

## Description of Preferred Embodiments

### Reactions

The reactions involved in the process of the present invention can be illustrated by the following equations:

Ester Formation

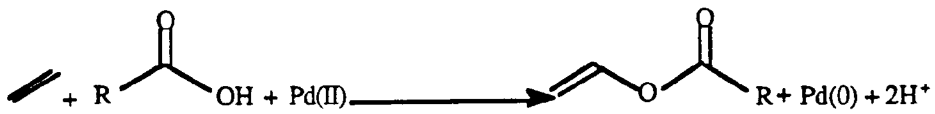

(A)

Palladium Oxidation:

$$Pd(0) + 2Cu(II) \rightarrow Pd(II) + 2Cu(I) \quad (B)$$

Copper Oxidation:

$$2Cu(I) + 1/2\ O_2 + 2H^+ \rightarrow 2Cu(II) + H_2O \quad (C)$$

Overall reaction:

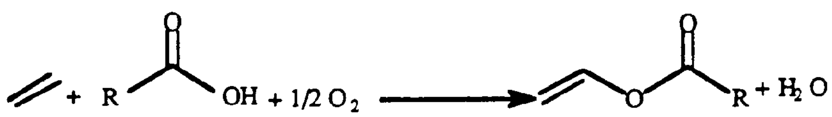

(D)

**Higher Carboxylic Acids**

The carboxylic acids employed in the process of the present invention generally include all carboxylic acids that contain at least four (preferably at least five or six) carbon atoms. Such carboxylic acids can be aliphatic or aromatic, cyclic, linear or branced and/or saturated or unsaturated. Illustrative of such carboxylic acids are butyric acid, crotonic acid, adipic acid, pivalic acid, 2-ethylhexanoic acid, neodecanoic acid and benzoic acid. Mixtures of such acids can be employed.

**Palladium Catalysts**

The palladium (II) catalysts employed in the process of the present invention are palladium compounds that contain palladium(II) and such compounds can be used as such in forming the reaction mixtures used in the process. Alternatively, a compound that generates palladium(II) in the reaction mixture can used in forming the reaction mixture. Such compounds that generate palladium(II) in the reaction mixture are referred to herein as "catalyst precursors". Illustrative of such catalysts are palladium(II) halides and palladium(II) carboxylates, preferably complexed with ligands. Illustrative of such catalyst precursors are palladium (0)-phosphine complexes such as palladium(tetrakistriphenyl-phosphine), palladium (0) complexes such as palladium bis-(dibenzylideneacetone) and palladium (0) colloids such as a palladium (0) colloid stabilized with polyvinyl pyrrolidone. The palladium (0) in such catalyst precursors is oxidized to palladium(II) by the copper (II) oxidant under reaction conditions employed in the process of the present invention (see equation (B) above). Palladium(II) acetate, palladium(II) 2-ethylhexanoate and bis(benzonitrile)palladium(II) dichloride are particularly useful as catalysts. Best results were obtained using palladium (0) colloid stabilized with polyvinylpyrrolidone as a catalyst precursor.

During the practice of the process of the present invention, palladium precipitation may occur. Palladium precipitation can be minimized or prevented by carrying out the process of the present invention using, as the catalyst precursor, a palladium (0) colloid. Such colloids are mixtures containing a minor amount of palladium (0) and a minor amount of a stabilizer dispersed in a major amount of an organic medium (e.g., the higher carboxylic acid reactant alone or mixed with an organic solvent). The stabilizer is an organic polymer containing polar functional groups such as amide groups, (i.e., any

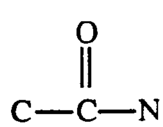 or 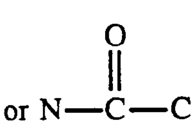

group regardless of further substitution), ketone groups, (i.e., any

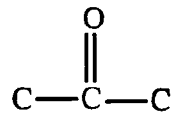

group regardless of further substitution), carbamate groups, (i.e., any

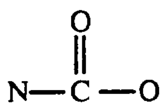 or 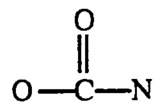

group regardless of further substitution), urea groups, (i.e., any

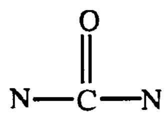

group regardless of further substitution) and carbonate groups, (i.e., any

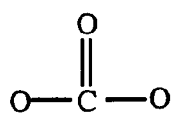

group regardless of further substitution).

The organic polymer stabilizers which are useful in such colloids are well known compounds and methods for their preparation are also well known. Any organic polymer, including homopolymers, copolymers, terpolymers and oligomers containing such polar functional groups may be used as stabilizers in such colloids. The polar functional groups may be present in the organic polymers as substituents pendant from the backbone of the polymer and/or as radicals that are incorporated in and form part of the backbone of the polymer. Further, the polar functional groups may be of a non-cyclic nature or part of a cyclic radical. The organic polymers may contain only one type of such polar functional groups or two or more different types of such polar functional groups. Illustrative organic polymers containing such polar functional groups that are employable in this invention include e.g. polyvinylpyrollidone, vinylpyrrolidone-vinyl acetate copolymers, polyacrylamides, copolymers of vinylpyrrolidone and beta-dimethylaminoethyl methacrylate, carbamic acid, N-[polymethylene(-polyphenyl)]methylester, N[polymethylene(polyphenyl)]N'-diisopropyl urea, copolymers of vinyl pyrrolidone and long chain alpha olefins, copolymers of vinyl pyrrolidone and styrene, polyacrylic acid hydrazide, poly-N-vinyl-5-methoxazolidone, polypeptides, e.g. poly-L-pyroline and poly-L-phenylalanine, and the like.

The amount of such organic polymer stabilizers in the palladium (0) colloids employable in the process of the present invention need only be that minimum amount necessary to reduce palladium precipitation that might be found to occur as a result of carrying out the process under identical conditions, save for the absence of the stabilizer. Amounts of such organic polymer stabilizers ranging from about 200 up to about 2000 weight percent based on the weight of the palladium are generally useful. The colloids can contain from $2x10^{-5}$ to 0.05 weight percent of palladium (0) based on the weight of the organic medium.

Such palladium (0) colloids are generally produced by the following process: a palladium(II) compound dissolved in an alcohol is reduced to palladium (0) by a base in the presence of an organic polymer stabilizer. The alcohol is evaporated and the palladium (0) and the stabilizer are redispersed in the desired organic medium to form the colloid.

**Palladium Catalyst Concentration**

As is shown in equation (B) above, palladium(II) activity is maintained by oxidizing palladium (0) {i.e., spent palladium formed in accordance with equation (A) above) to palladium (II) using a copper(II) oxidant. For this purpose, at least one equivalent of copper(II) oxidant per equivalent of palladium (0) is used. When a catalyst

precursor is used, the palladium (0) in the catalyst precursor is similar to the palladium (0) in the spent catalyst in as much as the palladium (0) from both sources is oxidized to palladium(II) by the copper(II) oxidant.

The concentration of the palladium(II) catalyst employed in the process of the present invention must be high enough so that sufficient catalytic activity is obtained. However, at too high concentrations, palladium may precipitate as the metal, especially at higher temperatures. Palladium metal is catalytically inactive in the present process. Further, too high palladium concentrations may require such large amounts of copper oxidant in order for the palladium to remain catalytically active that the solubility limit of the copper (II) oxidant in the reaction mixture may be exceeded. Such insolubility should be avoided because the copper (II) oxidant must remain in solution in the reaction mixture in to oxidize the palladium (0). In general, suitable palladium (II) catalyst concentration ranges (in moles of catalyst per liter of reaction mixture) are:

| | Ranges | |
|---|---|---|
| | Broad | Preferred |
| High limit | $5X10^{-2}$ | $5X10^{-3}$ |
| Low limit | $1X10^{-5}$ | $2X10^{-4}$ |

## Copper Oxidants

The copper (II) oxidants employed in the process of the present invention are copper complexes which function as oxidants for palladium(0) and which are soluble in the reaction mixture. Illustrative of such oxidants are copper (II) carboxylates of higher esters that are soluble in organic solvents, such as copper (II) 2-ethylhexanoate, copper(II) butyrate, copper(II) neodecanoate, copper(II) acetate and copper(II) benzoate. Copper(II) 2-ethylhexanoate gave the best catalyst activity and stability. The afore-mentioned specific copper complexes were found to me more effective than copper(II) chloride. Copper(II) gluconate, copper(II) acetoacetate, copper(II) acetylacetonate, and copper (II) hydroxide were found to be ineffective.

In one embodiment of the process of the present invention, the copper (II) oxidant is a copper (II) carboxylate in which carboxylate group has a molecular weight that is higher than the molecular weight of the carboxylate group in the carboxylic acid reactant. In this embodiment, a noticeable improvement in catalytic activity is achieved. By way of illustration, copper(II) 2-ethylhexanoate can be used as the oxidant when the acid reactant is butyric acid, crotonic acid, adipic acid, pivalic acid or benzoic acid.

## Copper Oxidant Concentration

The concentration of the copper (II) oxidant employed in the process of the present invention must be at least equal (on a mole basis) to the palladium concentration. The copper (II) oxidant oxidizes the palladium(0) in the catalyst precursor (if a precursor is employed) and also oxidizes the palladium(0) as it is formed from palladium(II) during the ester-forming reaction [see equations (A) and (B) above]. However, the activity of the oxidants appears to level off at high copper concentrations for a given palladium concentration. For example, at $2X10^{-3}$ molar palladium, activity for vinyl 2-ethylhexanoate synthesis at 110°C increases with copper concentration up to about 0.3 molar copper and then remains constant. The copper (II) oxidant concentration should not be so large that the solubility limit of the oxidant in the reaction mixture is exceeded. Suitable oxidant concentration ranges (in moles of oxidant per liter of reaction mixture) are:

| | Ranges | |
|---|---|---|
| | Broad | Preferred |
| High limit | 1.0 | 0.5 |
| Low limit | $1X10^{-5}$ | 0.2 |

## Reaction Temperature

The reaction temperature employed in the process of the present invention should be sufficiently high so

that acceptable catalyst activity is maintained but low enough so that the palladium does not precipitate from the reaction mixture. Undesirable palladium precipitation is more likely to occur at higher palladium concentrations and so the reaction temperature should be chosen considering the palladium concentration. The reaction temperature should also be such that the catalyst and the reactants (particularly the carboxylic acid reactant) are maintained in the homogeneous liquid phase. The choice of the reaction temperature is also determined by the ratio of ethylene and oxygen used in the process. Preferably the gas mixture should remain non-flammable. Suitable reaction temperatures are:

| | Ranges | |
|---|---|---|
| | Broad | Preferred |
| High limit | 200°C | 130°C |
| Low limit | 50°C | 95°C |

**Partial Pressure of Ethylene**

The relative amounts of ethylene and oxygen (as such or mixed with an inert gas such as nitrogen when air is used) employed in the process of the present invention should preferably be chosen so that the gas mixture is non-flammable under the reaction conditions. Sufficient ethylene should be added that an acceptable level of catalyst activity is maintained. Most reactions in the Examples appearing below were run with a non-flammable gas mixture at 300 psi ethylene and 200 psi air. Suitable ethylene partial pressures are:

| | Ranges (psig) | |
|---|---|---|
| | Broad | Preferred |
| High limit | 2000 | 500 |
| Low limit | 50 | 50 |

**Partial Pressure of Oxygen**

Again, the relative amounts of ethylene and oxygen should preferably be chosen so that the mixture is non-flammable under the reaction conditions. No improvement in activity was noted on substituting a corresponding amount of pure oxygen for air. However, for a continuous process, it will be more convenient to employ pure oxygen. The partial pressure of oxygen is more critical than the partial pressure of ethylene in that sufficient oxygen must be present to rapidly oxidize copper (I) as it is formed in solution back to copper (II). The copper(II) in turn oxidizes the palladium(0) (that is in the catalyst precursor or that is in spent catalyst) to palladium(II) so that the palladium will not precipitate from solution and will remain in the reaction mixture in its catalytically active form. Suitable oxygen partial pressures are:

| | Ranges (psig) | |
|---|---|---|
| | Broad | Preferred |
| High limit | 2000 | 500 |
| Low limit | 50 | 50 |

**Total Pressure**

The total pressure employed in the process of the present invention is largely determined by the ethylene and oxygen partial pressures. Suitable total pressures are:

| | Ranges (psig) | |
|---|---|---|
| | Broad | Preferred |
| High limit | 2000 | 500 |
| Low limit | 50 | 50 |

## Reaction Solvent

A solvent is employed in the process of the present invention to dissolve the palladium (II) catalyst, the copper (II) oxidant and the higher carboxylic acid reactant so as to achieve a homogeneous reaction mixture. The higher carboxylic reactants themselves, if they are liquids at 25°C (such as 2-ethylhexanoic acid, butyric acid and crotonic acid), are suitable solvents. If the higher carboxylic acid reactant is a solid at 25°C, an organic solvent can be used. Useful organic solvents are oxygenated solvents such as tertiary-butanol, tertiary-amyl alcohol, methyl propasol and tetrahydrofuran (THF). In a continuous process, a portion of the higher vinyl ester product may be used as the solvent.

## Promoters

Lithium chloride may be used as a catalyst promoter in the process of the present invention. The concentration of the lithium chloride promoter, if any, employed in the process of the present invention should be higher than the concentration of the palladium(II) catalyst. However, at sufficiently high concentrations, the lithium chloride becomes inhibitory and the catalyst activity decreases. For example, at $2X10^{-3}$ molar palladium and 0.4 molar copper 2-ethylhexanoate, the activity for vinyl 2-ethylhexanoate synthesis increased up to 0.1 molar lithium chloride. At higher lithium chloride concentrations, the catalyst activity decreased. Suitable promoter concentrations (in moles of promoter per liter of the reaction mixture) are:

| | Ranges | |
|---|---|---|
| | Broad | Preferred |
| High limit | 1.0 | 0.4 |
| Low limit | $1X10^{-5}$ | 0.05 |

When lithium chloride is used as a promoter, it is desirable to "precondition" the reaction vessel by washing the reaction vessel with the lithium chloride so as to increase the yield of the higher vinyl ester.

A polyglyme compound may also be used as catalyst promoter in the process of the present invention. Such compounds have the formula:

$$CH_3O(CH_2CH_2O)_nCH_3$$

where n has a value of at least two (preferably 2, 3 or 4). The amount of any polyglyme promoter used may be from 10 to 50 weight percent (and is preferably from 20 to 40 weight percent) based on the total weight of the reaction mixture. Larger amounts of polyglyme should not be used with palladium (0) colloids because the colloids may precipitate. An advantage of using a polyglyme promoter is that catalyst activity is increased.

## Separation Of Product

The higher vinyl esters produced by the process of the present invention can be separated from the other components of the reaction mixture by any suitable method. Thus, the water produced as a byproduct in the process can be separated from the higher vinyl ester by volatilization. The palladium (II) catalyst and copper (II) oxidant can be separated from the higher vinyl ester by volatilization. Other separation methods include distillation, membrane filtration, wiped-film evaporation and ultracentrifugigation.

## Ester Products

The higher vinyl esters produced by the process of the present invention are known compounds having known utilities. The esters are particularly useful in producing polymers having higher glass transition temperatures different from the glass transition temperatures that can be obtained with vinyl acetate.

In the Examples appearing below, the abbreviations used have the following meanings:

| ABBREVIATION | MEANING |
|---|---|
| mL | milliliter |
| PVP | polyvinylpyrollidone |
| M | molar |
| g | grams |
| Temp. | temperature in °C |
| VEH | vinyl-2-ethylhexanoate |
| mol/1-hr. | moles per liter per hour |
| min | minute |
| hr | hour |
| psig or psi | pounds per square inch gauge |
| THF | tetrahydrofuran |

The following Examples illustrate the present invention.

**Example 1**

Synthesis of Palladium Colloid Stabilized with Polyvinylpyrrolidone

A 500 mL 3-neck round bottom flask was fitted with a water-cooled reflux condenser and charged with .69g $Na_2PdCl_4$, .88g "PVP-K15" (a polyvinylpyrrolidone stabilizer that is sold by the GAF Corporation.) and 384 mL methanol. The solution was refluxed for 30 minutes and then 16 mL of a 0.5M solution of NaOH in methanol was added. The solution turned dark brown immediately due to the formation of palladium (0). The methanol colloid so formed was refluxed 20 additional minutes and was then cooled to room temperature. In the Examples appearing below, colloids containing the palladium (0) and the stabilizer dispersed in carboxylic acids or other solvents were prepared by measuring out the appropriate amount of the methanol colloid, evaporating the methanol colloid to dryness on a rotary evaporator and then redispersing the palladium (0) and the stabilizer in the appropriate organic medium to the desired concentration as specified in the Examples.

In the following Examples, the yields of the higher vinyl ester products are calculated based on the total weight of acid present, including any acid generated from the copper (II) oxidant. By way of illustration, in producing vinyl 2-ethylhexanoate from a 2-ethylhexanoic acid reactant and a copper(II) 2-ethylhexanoate oxidant, the total 2-ethylhexanoic acid present is the sum of the amount of the acid added as a reactant and the amount of the acid that is liberated from the copper(II) 2-ethylhexanoate.

**Example 2**

Synthesis of Vinyl 2-ethylhexanoate Using Pd/Copper(II) 2-ethylhexanoate

Thirty mL of a palladium (0) colloid containing 2-ethylhexanoic acid as the organic medium (palladium (0) concentration .0002M), 4.32g copper(II) 2-ethylhexanoate (0.4 M), and 2.3g N-methylpyrrolidone (as an internal standard) was charged to a 100 mL Parr reactor. The reaction was heated to 110°C and pressurized to 300 psig with ethylene, then to 500 psi with air. The initial reaction rate was determined by sampling of the solution after 30 minutes and analysis by gas chromatography. The rate of reaction was found to be 0.14 mol/1-hr. After two hours of reaction the solution was analyzed by gas chromatography to determine product composition.

The major products were .98g vinyl 2-ethylhexanoate (2.5% yield) and .02g acetaldehyde. Table 1 below summarizes other reactions employing palladium colloid/copper 2-ethylhexanoate catalyst for vinyl 2-ethylhexanoate synthesis, showing the effects of Pd concentration, copper concentration, and temperature. All reactions reported in Table 1 were conducted under 300 psi ethylene and 200 psi air.

## Table 1

| Pd, M | Cu, M | Temp. | Total Pressure | Rate | VEH, g | $CH_3CHO$, g | Yield |
|---|---|---|---|---|---|---|---|
| .0002 | 0.4 | 110° | 500 | .14 | .98 | .02 | 2.5% |
| .0002 | 0.2 | 110° | 500 | .10 | .93 | .02 | 2.5% |
| .0002 | 0.1 | 110° | 500 | .10 | 1.1 | .04 | 2.7% |
| .0002 | 0.05 | 110° | 500 | .06 | 0.7 | .02 | 1.8% |
| .0002 | 0.01 | 110° | 500 | .03 | 0.3 | – | 0.8% |
| .002 | 0.4 | 110° | 500 | .52 | 3.1 | * | 7.8% |
| .002 | 0.4 | 95° | 500 | .38 | 2.1 | .31 | 5.3% |
| .002 | 0.4 | 130° | 500 | .44 | 2.7 | .06 | 6.8% |

* Not determined

The results in Table 1 above show that yield of vinyl 2-ethylhexanoate generally increases with temperature and palladium catalyst concentration, but that for a given palladium concentration the effect of copper concentration becomes constant above a certain value.

The following Example illustrates the use of semi-continuous oxygen addition to avoid catalyst precipitation and increase catalyst rate, productivity, and lifetime.

### Example 3

Synthesis of Vinyl 2-ethylhexanoate Using Fd/Copper(II) 2-ethylhexanoate, and Semi-Continuous Air Addition

Thirty mL of a palladium (0) colloid containing 2-ethylhexanoic acid as the organic medium (palladium (0) concentration .002M), 4.32g copper(II) 2-ethylhexanoate (0.4 M), and 3.1 g N-methylpyrrolidone (as an internal standard) was charged to a 100 mL Parr reactor. The reaction was heated to 110°C and pressurized to 300 psig with ethylene, then to 500 psi with air. Every 30 minutes, the reaction mixture was sampled for analysis by gas chromatography, the atmosphere was vented to purge the system of built-up nitrogen and then the reactor was freshly recharged with 300 psi ethylene, 200 psi air, and then the mixture was sampled again. This procedure allowed the determination of the amount of product formed in each 30 minute reaction. After two hours total reaction time, the solution was analyzed by gas chromatography to determine the final product composition. The initial reaction rate was .5 mol/1-hr and the final product mixture contained 5.8g vinyl 2-ethylhexanoate (15% yield). Little acetaldehyde was detected in the final product, having been purged from the headspace vapor during venting and repressurizing. The Table 2 below summarizes the data obtained during this reaction:

**Table 2**

| | Vinyl 2-ethylhexanoate | Acetaldehyde |
|---|---|---|
| First product | 1.33g | .06g |
| Second charge | 1.53g | .05g |
| Second product | 2.88g | .08g |
| Third charge | 3.30g | .07g |
| Third product | 4.40g | .10g |
| Fourth charge | 4.09g | -- |
| Fourth product | 5.82g | .02g |

The data in Table 3 illustrates the synthesis of vinyl 2-ethylhexanoate by this seem-continuous method and the effect of palladium and copper concentration and reaction temperature. All these reactions were run at 300 psi ethylene and 200 psi air. The amount of vinyl 2-ethylhexanoate after the initial 30 minute reaction and after 2 hours total reaction time is shown in Table 3 below.

**Table 3**

| Pd, M | Cu, M | Temp. | Total Pressure | Rate | VEH, g (30 min) | VEH, g (2 hr) | Yield |
|---|---|---|---|---|---|---|---|
| .002 | 0.4 | 110° | 500 | .49 | 1.33 | 5.82 | 15% |
| .002 | 0.4 | 120° | 500 | .61 | 1.66 | 4.82 | 12% |
| .002 | 0.4 | 130° | 500 | .75 | 2.05 | 6.28 | 16% |
| .002 | 0.1 | 110° | 500 | .12 | 0.77 | 2.64 | 7% |
| .002 | 0.2 | 110° | 500 | .33 | 0.92 | 2.98 | 8% |

The data in Table 3 show that, although the initial rate and amount of vinyl 2-ethyl hexanoate formation increases with temperature, at temperatures above 110° or at copper concentrations below 0.4 molar, the final amount of vinyl 2-ethylhexanoate produced is lower than expected. This is believed to be due to the decomposition of the palladium catalyst at higher temperatures and lower copper concentrations where reoxidation of palladium(0) is less efficient. It is expected that more frequent or continuous addition of oxygen will prevent or slow this decomposition.

The following Example illustrates the use of copper chloride and copper acetate, oxidants for palladium(0) frequently cited in the prior art, instead of copper(II) 2-ethylhexanoate. This Example shows that lower efficiency to vinyl esters is obtained using these oxidants compared to copper 2-ethylhexanoate under identical conditions.

**Example 4**

Synthesis of Vinyl 2-ethylhexanoate Using Pd/$CuCl_2$ Catalyst

Thirty mL of a palladium (0) colloid containing 2-ethylhexanoic acid as the organic medium (palladium (0) concentration .002M), 2.04g copper (II) chloride (0.4M), and 2.2g N-methylpyrrolidone (as an internal standard) was charged to a 100 mL Parr reactor. The reaction was heated to 110°C and pressurized to 300 psig with ethylene and then pressurized to 500 psi with air. The reaction was vented, sampled and repressurized every 30 minutes for two hours as in the previous Examples. After two hours reaction time the product mixture was determined by gas chromatography to contain .34g vinyl 2-ethylhexanoate (1% yield) and.2g acetaldehyde.

**Example 5**

Synthesis of Vinyl 2-ethylhexanoate Using Pd/Cu(OAc)2 Catalyst

Thirty mL of a palladium(0) colloid containing 2-ethylhexanoic acid as the organic medium (palladium(0) concentration .200M), 2.32g copper(II) acetate (0.4M), and 2.3g N-methylpyrrolidone (as an internal standard) was charged to a 100 mL Parr reactor. The reaction was heated to 110°C and pressurized to 300 psig with ethylene and then pressurized to 500 psi with air. The reaction was vented, sampled, and repressurized every 30 minutes for two hours as in the previous Examples. After two hours reaction time the product mixture was determined by gas chromatography to contain 0.71g vinyl 2-ethylhexanoate (2% yield) and traces of acetaldehyde.

The following Comparative Example illustrates that addition of copper acetate to a palladium colloid/copper 2-ethylhexanoate catalyst solution results in a reduced yield of vinyl 2-ethylhexanoate.

**Comparative Example 6**

Synthesis of Vinyl 2-ethylhexanoate Using Pd/Cu(2-EH)$_2$/Cu(OAc)$_2$ Catalyst

A reaction mixture similar to that of Example 3 above, but also containing .58g copper(II) acetate (0.1M), was charged to a 100 mL Parr reactor. The reaction was heated to 110°C and pressurized to 300 psig with ethylene, and then pressurized to 500 psi with air. The reaction was vented, sampled and repressurized every 30 minutes for two hours as in the previous Examples. After two hours reaction time, the product mixture was determined by gas chromatography to contain 2.54g vinyl 2-ethylhexanoate, compared with 5.82g obtained using copper 2-ethylhexanoate alone.

Examples 7 to 9 below illustrate that palladium complex precursors may be used instead of a palladium(0) colloid for vinyl 2-ethylhexanoate synthesis.

**Example 7**

Synthesis of Vinyl 2-ethylhexanoate Using Palladium(II) 2-ethylhexanoate Catalyst

A catalyst solution consisting of .071g palladium(II) 2-ethylhexanoate dissolved in 2-ethylhexanoic acid (palladium(II) concentration .002M), 4.30g copper(II) 2-ethylhexanoate (0.4M), 2.3g N-methylpyrrolidone (as an internal standard), .06g polyvinylpyrrolidone-vinylacetate copolymer, and 30 mL 2-ethylhexanoic acid was charged to a 100 mL Parr reactor. The reaction was heated to 110°C and pressurized to 300 psig with ethylene, and then pressurized to 500 psi with air. The reaction mixture was vented, sampled and repressurized every thirty minutes as in previous examples. After two hours total reaction time, the solution was analyzed by gas chromatography to determine the final product composition. The initial reaction rate was .4 mol/1-hr and the final product mixture contained 4.6g vinyl 2-ethylhexanoate (12% yield).

**Example 8**

Synthesis of Vinyl 2-ethylhexanoate Using Bis(benzonitrile)Palladium(II) Dichloride Catalyst

A catalyst solution consisting of .024g bis(benzonitrile)palladium(II) dichloride (.002M), 4.33g copper(II) 2-ethylhexanoate (0.4M), 2.3 g N-methylpyrrolidone (as an internal standard), .14g lithium chloride and 30 mL 2-ethylhexanoic acid was charged to a 100 mL Parr reactor. The reaction was heated to 110°C and pressurized to 300 psig with ethylene and then pressurized to 500 psi with air. The reaction mixture was vented, sampled, and repressurized every thirty minutes as in previous Examples. After two hours total reaction time, the solution was analyzed by gas chromatography to determine the final product composition. The initial reaction rate was .8 mol/1-hr and the final product mixture contained 7.5 g vinyl 2-ethylhexanoate (19% yield) and .24g acetaldehyde.

**Example 9**

Synthesis of Vinyl 2-ethylhexanoate Using Palladium(II) Acetate Catalyst

A catalyst solution consisting of .0014g palladium(II) acetate (palladium(II) concentration .0002M), 2.15g copper 2-ethylhexanoate (0.2 M), 2.3 g N-methylpyrrolidone (as an internal standard), and 30 mL 2-ethylhexanoic acid was charged to a 100 mL parr reactor. The reaction was heated to 110°C and pressurized to 300 psig with ethylene and then pressurized to 500 psi with air. The reaction mixture was vented, sampled, and repressurized after one hour. After two hours total reaction time, the solution was analyzed by gas chromatography to determine the final product composition. The initial reaction rate was .1 mol/1-hr and the final product mixture contained 1.27g vinyl 2-ethylhexanoate (3% yield) and .06g acetaldehyde.

Examples 10 and 11 below illustrate that the process of this invention may be carried out in an organic solvent.

**Example 10**

Synthesis of Vinyl 2-ethylhexanoate in Tertiary-amyl Alcohol Solvent.

A palladium (0) colloid containing 10 mL 2-ethylhexanoic acid and 20 mL t-amyl alcohol as the organic medium (palladium(0) concentration .002M), 4.33g copper(II) 2-ethylhexanoate (0.4 M),.13g lithium chloride, and 2.3 g N-methylpyrrolidone (as an internal standard) was charged to a 100 mL Parr reactor. The reaction was heated to 110°C and pressurized to 300 psig with ethylene and then pressurized to 500 psi with air. The reaction mixture was vented, sampled, and repressurized every thirty minutes as in previous examples. After two hours total reaction time, the solution was analyzed by gas chromatography to determine the final product composition. The initial reaction rate was .8 mol/1-hr and the final product mixture contained 8.8 g vinyl 2-ethylhexanoate (55% yield) and .23g acetaldehyde.

**Example 11**

Synthesis of Vinyl 2-ethylhexanoate in Tertiary-amyl Alcohol Solvent

A palladium (0) colloid containing 5 mL 2-ethylhexanoic acid and 25 mL t-amyl alcohol as the organic medium (palladium (0) concentration .002M), 4.33g copper(II) 2-ethylhexanoate (0.4M), .13 g lithium chloride, and 2.4 g N-methylpyrrolidone (as an internal standard) was charged to a 100 mL Parr reactor. The reaction was heated to 110°C and pressurized to 300 psig with ethyleneand then pressurized to 500 psi with air. The reaction mixture was vented, sampled, and repressurized every thirty minutes as in previous examples. After two hours total reaction time, the solution was analyzed by gas chromatography to determine the final product composition. The initial reaction rate was .8 mol/1-hr and the final product mixture contained 7.0 vinyl 2-ethylhexanoate (69% yield) and .11g acetaldehyde.

Examples 12 to 21 below illustrate the synthesis of various vinyl carboxylates using palladium colloid/copper 2-ethylhexanoate catalyst and the synthesis of vinyl esters from solid, higher carboxylic acids in an organic solvent.

**Example 12**

Synthesis of Vinyl Butyrate Using Pd/copper(II) 2-ethylhexanoate Catalyst

A palladium (0) colloid containing 30 mL butyric acid as the organic medium (palladium (0) concentration .002 M), 2.19g copper(II) 2-ethylhexanoate (0.2M), and 2.2g N-methylpyrrolidone (as an internal standard) was charged to a 100 mL Parr reactor. The reaction was heated to 110°C and pressurized to 300 psi with ethylene and then pressurized to 500 psi with air. After two hours reaction time, the mixture was analyzed by gas chromatography to determined product composition. Approximately 1 gram vinyl butyrate was produced. 2-Ethylhexanoic acid was also generated in solution from exchange of butyric acid with copper(II) 2-ethylhexanoate, producing 0.1g vinyl 2-ethylhexanoate in the reaction.

**Example 13**

Synthesis of Vinyl Crotonate Using Pd/copper(II) 2-ethylhexanoate Catalyst in Tertiary-butanol Solvent

A palladium (0) colloid containing 15 mL crotonic acid and 15 mL t-butanol as the organic medium (palladium (0) concentration .002M), 4.20g copper(II) 2-ethylhexanoate (0.4M), and 2.8g N-methylpyrrolidone (as an internal standard) was charged to a 100 mL Parr reactor. The reaction was heated to 110°C and pressurized to 300 psig with ethylene and then pressurized to 500 psi with air. The reaction mixture was vented, sampled, and repressurized every thirty minutes as in previous examples. After two hours total reaction time, the solution was analyzed by gas chromatography to determine the final product composition. The initial reaction rate was .5 mol/1-hr and the final product mixture contained 2.0g vinyl crotonate and an unquantified amount of vinyl 2-ethylhexanoate.

**Example 14**

Synthesis of Vinyl Crotonate Using Pd/copper(II) 2-ethylhexanoate Catalyst in Tertiary-amyl Alcohol Solvent

A palladium (0) colloid containing 10g crotonic acid and 20 mL t-butanol as the organic medium (palladium (0) concentration .002M), 4.34g copper(II) 2-ethylhexanoate (0.4 M), and 2.4 g N-methylpyrrolidone (as an internal standard) was charged to a 100 mL Parr reactor. The reaction was heated to 100°C and pressurized to 300 psig with ethylene and then pressurized to 500 psi with air. The reaction mixture was vented, sampled, and repressurized every thirty minutes as in previous Examples. After two hours total reaction time, the solution was analyzed by gas chromatography to determine the final product composition. The initial reaction rate was .3 mol/1-hr and the final product mixture contained 2.8g vinyl crotonate, .24g acetaldehyde, and an unquantified amount of vinyl 2-ethylhexanoate.

**Example 15**

Synthesis of Vinyl Crotonate Using Pd/copper(II) 2-ethylhexanoate Catalyst in Tetrahydrofuran Solvent

A palladium (0) colloid containing 10g crotonic acid and 20 mL THF as the organic medium (palladium (0) concentration .002M), 4.31g copper(II) 2-ethylhexanoate (0.4 M), and 2.2 g N-methylpyrrolidone (as an internal standard) was charged to a 100 mL Parr reactor. The reaction was heated to 110°C and pressurized to 300 psig with ethylene and then pressurized to 500 psi with air. The reaction mixture was vented, sampled, and repressurized every thirty minutes as in previous examples. After two hours total reaction time, the solution was analyzed by gas chromatography to determine the final product composition. The initial reaction rate was .4 mol/1-hr and the final product mixture contained 2.2g vinyl crotonate, .21g acetaldehyde, and an unquantified amount of vinyl 2-ethylhexanoate.

**Example 16**

Synthesis of Vinyl Crotonate Using Pd/copper(II) 2-ethylhexanoate Catalyst in Methyl Propasol Solvent

A palladium (0) colloid containing 10g crotonic acid and 20 mL methyl propasol as the organic medium (palladium (0) concentration .002M), 4.30g copper(II) 2-ethylhexanoate (0.4M), and 2.3 g N-methylpyrrolidone (as an internal standard) was charged to a 100 mL Parr reactor. The reaction was heated to 110°C and pressured to 300 psig with ethylene and then pressurized to 500 psi with air. The reaction mixture was vented, sampled, and repressurized every thirty minutes as in previous examples. After two hours total reaction time, the solution was analyzed by gas chromatography to determine the final product composition. The initial vinyl crotonate formation rate was .6 mol/1-hr and the final product mixture contained 2.7g vinyl crotonate,.33g acetaldehyde, and .88 vinyl 2-ethylhexanoate.

**Example 17**

Synthesis of Vinyl Pivalate Using Pd/copper(II)2-ethylhexanoate Catalyst in Tertiary-butanol Solvent

A palladium (0) colloid containing 20g pivalic acid and 10 mL t-butanol as the organic medium (palladium (0) concentration .002M), 4.2g copper(II) 2-ethylhexanoate (0.4M), and 2.4 g N-methylpyrrolidone (as an internal standard) was charged to a 100 mL Parr reactor. The reaction was heated to 110°C and pressured to 300 psig with ethylene and then pressurized to 500 psi with air. The reaction mixture was vented, sampled, and repressurized every thirty minutes as in previous examples. After two hours total reaction time, the solution was analyzed by gas chromatography to determine the final product composition. The initial vinyl pivalate formation rate was .2 mol/1-hr and the final product mixture contained 1.3g vinyl pivalate,.02g acetaldehyde, and an unquantified amount of vinyl 2-ethylhexanoate.

**Example 18**

Synthesis of Vinyl Neodecanoate Using Pd/copper(II) 2-ethylhexanoate Catalyst in Tertiary-butanol Solvent

A palladium (0) colloid containing 20g acid and 10 mL t-butanol as the organic medium (palladium (0) concentration .002M), 2.2g copper(II) 2-ethylhexanoate (0.2M), and 2.4 g N-methylpyrrolidone (as an internal standard) was charged to a 100 mL Parr reactor. The reaction was heated to 110°C and pressurized to 300 psig with ethylene and then pressurized to 500 psi with air. The reaction mixture was vented, sampled, and repressurized every thirty minutes as in previous examples. After two hours total reaction time, the solution was analyzed by gas chromatography to determine the final product composition. The final product mixture contained .6g vinyl neodecanoate and unquantified amounts of acetaldehyde and 2-ethylhexanoic acid.

**Example 19**

Synthesis of Vinyl Benzoate Using Pd/copper(II) 2-ethylhexanoate Catalyst in Tertiary-butanol Solvent

A palladium colloid containing 10g benzoic acid and 20 mL t-butanol as the organic medium (palladium (0) concentration .002M), 4.3g copper(II) 2-ethylhexanoate (0.4M), and 2.2 g N-methylpyrrolidone (as an internal standard) was charged to a 100 mL Parr reactor. The reaction was heated to 110°C and pressurized to 300 psig with ethylene and then pressurized to 500 psi with air. The reaction mixture was vented, sampled, and

repressurized every thirty minutes as in previous examples. After two hours total reaction time, the solution was analyzed by gas chromatography to determine the final product composition. The initial rate of formation of vinyl benzoate was .5 mol/1-hr, and the final product mixture contained 2.6g vinyl benzoate (21% yield), .1g acetaldehyde, and an unquantified amount of 2-ethylhexanoic acid. This is the best result obtained in these Examples for vinyl benzoate synthesis.

**Example 20**

Synthesis of Vinyl Adipates Using Pd/copper(II) 2-ethylhexanoate in Tertiary-butanol Solvent

A palladium (0) colloid containing in 10g adipic acid and 20 mL t-butanol as the organic medium (palladium (0) concentration .002M), 4.3g copper(II) 2-ethylhexanoate (0.4M), and 2.5g N-methylpyrrolidone (as an internal standard) was charged to a 100 mL Parr reactor. The reaction was heated to 110°C and pressurized to 300 psig with ethylene and then pressurized to 500 psi with air. The reaction mixture was vented and repressurized every thirty minutes as in previous examples. After two hours total reaction time, the solution was analyzed by gas chromatography to determine the final product composition. The final product mixture contained 5.1g monovinyl adipate (44% yield),.3g divinyl adipate (2% yield), and unquantified amounts of acetaldehyde and 2-ethylhexanoic acid. This is the best result obtained in these Examples for vinyl adipates synthesis.

**Example 21**

Synthesis of Vinyl Adipates and Vinyl 2-ethylhexanoate Using Pd/copper(II) 2-ethylhexanoate in 2-ethylhexanoic Acid Solvent

A palladium (0) colloid containing 20mL 2-ethylhexanoic acid as the organic medium (palladium (0) concentration .002M), 4.3g copper(II) 2-ethylhexanoate (0.4M), and 2.4g N-methylpyrrolidone (as an internal standard) was charged to a 100 mL Parr reactor. The reaction was heated to 110°C and pressurized to 300 psig with ethylene and then pressurized to 500 psi with air. The reaction mixture was vented and repressurized every thirty minutes as in previous examples. After two hours total reaction time, the solution was analyzed by gas chromatography to determine the final product composition. The average rate of monovinyl adipate formation was .2 mol/1-hr and the final product mixture contained 2.6 g monovinyl adipate,.3g divinyl adipate, 2.5 g vinyl 2-ethylhexanoate, and an unquantified amount of acetaldehyde.

Examples 22 to 25 below illustrate that addition of lithium chloride as a promoter increases the reaction rate and yield of higher vinyl esters.

**Example 22**

Synthesis of Vinyl 2-ethylhexanoate Using Pd/$Cu(2\text{-}EH)_2$/LiCl Catalyst

A reaction mixture similar to that of Example 3 above, but also containing .13g lithium chloride (0.1 M), as a promoter was charged to a 100 mL Parr reactor. The reaction was heated to 110°C and pressurized to 300 psig with ethylene and then pressurized to 500 psi with air. The reaction was vented, sampled, and pressurized every 30 minutes for two hours as in the previous Examples. The initial reaction rate was determined to be 0.7 mol/1-hr. After two hours reaction time, the product mixture was determined by gas chromatography to contain 8.27g vinyl 2-ethylhexanoate (23% yield) and .15g acetaldehyde, compared with 5.82g obtained using copper 2-ethylhexanoate alone. The data obtained from this reaction are shown below:

| | Vinyl 2-ethylhexanoate | Acetaldehyde |
|---|---|---|
| 30 minutes | 1.99g | .04g |
| 1 hour | 3.69g | .06g |
| 90 minutes | 5.60g | .10g |
| 2 hours | 8.27g | .15g |

**Example 23**

Synthesis of Vinyl Crotonate Using Pd/copper(II) 2-ethylhexanoate/LiCl Catalyst

A palladium (0) colloid containing 15g crotonic acid and 30 mL methyl propasol as the organic medium (palladium (0) concentration .002M), 6.46g copper(II) 2-ethylhexanoate (0.4 M), 19g LiCl (.1 M), and 3.8 N-methylpyrrolidone (as an internal standard) was charged to a 100 mL Parr reactor. The reaction was heated to 110°C and pressurized to 300 psig with ethylene and then pressurized to 500 psi with air. The reaction mixture was vented and pressurized every thirty minutes, and sampled every hour. The initial reaction rate was 0.7 mol/1-hr. After two hours reaction time, the solution was determined by gas chromatography to contain 4.1g vinyl crotonate, 0.7g acetaldehyde, compared with 2.7g vinyl crotonate produced using copper 2-ethylhexanoate alone. After six hours total reaction time the solution was analyzed by gas chromatography to determine the final product composition. The final product mixture contained 6.3g vinyl crotonate, 1.5g acetaldehyde, and an unquantified amount of vinyl 2-ethylhexanoate. This is the best result obtained in these Examples for the synthesis of vinyl crotonate.

## Example 24

Synthesis of Vinyl Pivalate Using Pd/copper(II) 2-ethylhexanoate/LiCl Catalyst

A palladium (0) colloid containing 20g pivalic acid and 10 mL t-butanol as the organic medium (palladium (0) concentration .002M), 4.3g copper(II) 2-ethylhexanoate (0.4 M),.13g LiCl (.1 M), and 2.4 g N-methylpyrrolidone (as an internal standard) was charged to a 100 mL Parr reactor. The reaction was heated to 110°C and pressurized to 300 psig with ethylene and then pressurized to 500 psi with air. The reaction mixture was vented, sampled, and pressurized after thirty minutes. After one hour total reaction time, the solution was analyzed by gas chromatography to determine the final product composition. The initial vinyl pivalate formation rate was .4 mol/1-hr and the final product mixture contained 1.7g vinyl pivalate, and unquantified amounts of acetaldehyde and vinyl 2-ethylhexanoate, compared with Example 17 above, in which the initial rate was .2 mol/1-hr and 1.3 grams vinyl pivalate were formed in two hours.

## Example 25

Synthesis of Vinyl Neodecanoate Using Pd/copper(II) 2-ethylhexanoate/LiCl Catalyst

A palladium(II) colloid containing 10 mL neodecanoic acid and 20 mL methyl propasol as the organic medium (palladium (0) concentration .002M), 4.3g copper(II) 2-ethylhexanoate (0.4 M),.13g LiCl (.1 M), and 2.3 g N methylpyrrolidone (as an internal standard) was charged to a 100 mL Parr reactor. The reaction was heated to 110°C and pressurized to 300 psig with ethylene and then pressurized to 500 psig with air. The reactor mixture was vented, sampled, and repressurized after third minutes. After two hours total reaction time, the solution was analyzed by gas chromatography to determine the final product composition. The initial vinyl pivalate formation rate was .3 mol/1-hr and the final product mixture contained 4.2g vinyl neodecanoate (37% yield), .2g acetaldehyde and an unquantified amount of vinyl 2-ethylhexanoate, compared with example 33-DLP-33, in which only .6 grams vinyl neodecanoate were formed in two hours. This is the best results obtained in these Examples for the synthesis of vinyl nedecanoate.

The following Example illustrates that at high concentrations, lithium chloride can act as an inhibitor for the ester-forming reaction involved in the process of the present invention.

## Example 26

Synthesis of Vinyl 2-ethylhexanoate Using Pd/Cu(2 EH)$_2$/LiCl Catalyst

A reaction mixture similar to that of Example 3 above but also containing .52g lithium chloride (0.4 M), as a promoter was charged to a 100 mL Parr reactor. The reaction was heated to 110°C and pressurized to 300 psig with ethylene, then to 500 psi with air. The reaction was vented, sampled, and repressurized every 30 minutes for two hours as in the previous examples. The initial reaction rate was determined to be 0.7 mol/1-hr. After two hours reaction time the product mixture was determined by gas chromatography to contain 2.42g vinyl 2-ethylhexanoate and .13g acetaldehyde, compared with 5.82 vinyl 2-ethylhexanoate obtained using copper 2-ethylhexanoate alone, and with 8.27g obtained with an additional 0.1 M lithium chloride.

Examples 27 and 28 below illustrate that the addition of diglyme as apromoter to the reaction mixture used in the process of the present invention increases the rate and yield of vinyl esters. Similar reactions using relatively large amounts of diglyme were not successful due to the insolubility of the colloid.

## Example 27

Synthesis of Vinyl 2-ethylhexanoate Using Pd/Cu(2 EH)$_2$/LiCl Catalyst in 2-ethylhexanoic Acid/diglyme Solvent

A palladium (0) colloid containing 20 mL 2-ethylhexanoic acid and 10 mL diglyme as the organic medium

(palladium (0) concentration .002M), 4.3g copper(II) 2-ethylhexanoate (0.4M), .14g lithium chloride (0.1M) and 2.4g N- methylpyrrolidone (as an internal standard) was charged to a 100 mL Parr reactor. The reaction was heated to 110°C and pressurized to 300 psig with ethylene, then to 500 psi with air. The reaction was vented, sampled, and repressurized every 30 minutes for two hours as in the previous examples. The initial reaction rate was deterined to be 1.0 mol/1-hr. After two hours reaction time the product mixture was determined by gas chromatography to contain 9.60g vinyl 2-ethylhexanoate (35% yield) and .14g acetaldehyde, compared with 5.92g vinyl 2-ethylhexanoate obtained using copper 2-ethylhexanoate alone, and with 8.27 g obtained with an additional 0.1 M lithium chloride. This result is best result obtained in these Examples for vinyl 2-ethylhexanoate synthesis.

## Example 28

Synthesis of Vinyl Pivalate, Pd/Cu(2-EH)$_2$/LiCl Catalyst in t-amyl Alcohol/diglyme solvent

A palladium (0) colloid containing 10 mL t-amyl alcohol, 10 mL diglyme and 10g pivalic acid as the organic medium (palladium (0) concentration .002M), 4.3g copper(II) 2-ethylhexanoate (0.4M), .13g lithium chloride (0.1M), and 2.3g N-methylpyrrolidone (as an internal standard) was charged to a 100 mL Parr reactor. The reaction was heated to 110°C and pressurized to 300 psig with ethylene, then to 500 psi with air. The reaction was vented, sampled, and repressurized every 30 minutes for two hours as in the previous examples. The initial reactio rate was determine to be 0.6 mol/1-hr. After two hours reaction time the product mixture was determined by gas chromatography to contain 2.5g vinyl pivalate (19% yield),.26g acetaldehyde, and an unquantified amount of vinyl 2-ethylhexanoate, compared with 1.27g vinyl pivalate obtained using copper 2-ethylhexanoate alone. This result is the best result obtained in these Examples for vinyl pivalate synthesis.

Examples 29 to 35 below illustrate the use of various copper (II) compounds in an effort to achieve the oxidation of palladium (0) to palladium (II). Some of the compounds produced active vinyl ester catalysts and some did not. None of these compounds were superior to copper 2-ethylhexanoate as an oxidant.

## Example 29

Synthesis of Vinyl 2-ethylhexanoate Pd/Copper(II) Ethylacetoacetate Catalyst

Thirty mL of a palladium (0) colloid containing 2-ethylhexanoic acid as the organic medium (palladium (0) concentration .002M), 3.86g copper(II) ethylacetoacetate (0.4M), and 2.3g N-methylpyrrolidone (as an internal standard) was charged to a 100 mL Parr reactor. The reaction was heated to 110°C and pressurized to 300 psig with ethylene, then to 500 psi with air. The reaction was vented, sampled, and repressurized every 30 minutes as in the previous examples. The initial reaction rate was 0.2 mol/1-hr. After 90 minutes reaction time the product mixture was determined by gas chromatography to contain 1.19g vinyl 2-ethylhexanoate (3% yield) and traces of acetyldehyde.

## Example 30

Synthesis of Vinyl 2-ethylhexanoate Pd/Copper Gluconate Catalyst

Thirty mL of a palladium (0) colloid containing 2-ethylhexanoic acid as the organic medium (palladium (0) concentration .002M), 5.43g copper(II) gluconate (0.4M) and 2.3 g N-methylpyrrolidone (as an internal standard) was charged to a 100 mL Parr reactor. The reaction was heated to 110°C and pressurized to 300 psig with ethylene, then to 500 psi with air. The reaction was vented, sampled,and repressurized every 30 minutes as in the previous examples. After 90 minutes reaction time the product mixture was determined by gas chromatography to contain .23g vinyl 2-ethylhexanoate (<1 % yield). The presence of a large amount of solids in the catalyst dump solution suggested that copper gluconate was not soluble in 2-ethylhexanoic acid.

## Example 31

Synthesis of Vinyl 2-ethylhexanoate Pd Pd/Copper(II) Acetylacetonate Catalyst

A palladium (0) colloid containing 2-ethylhexanoic acid as the organic medium (palladium (0) concentration .0002M), 3.11 g copper acetylacetonate (0.4M), and 2.2 g N-methylpyrrolidone (as an internal standard) was charged to a 100 mL Parr reactor. The reaction was heated to 110°C and pressurized to 300 psig with ethylene, then to 500 psi with air. After two hours reaction time the product mixture was determined by gas chromatography to contain only traces of vinyl ester.

**Example 32**

Synthesis of Vinyl 2-ethylhexanoate Pd/Copper (II) Hydroxide Catalyst

A palladium (0) colloid containing 2-ethylhexanoic acid as the organic medium (palladium (0) concentration .0002M), .58g copper(II) hydroxide (0.2M), and 2.0 g N-methylpyrrolidone (as an internal standard) was charged to a 100 mL Parr reactor. The reaction was heated to 100°C and pressurized to 300 psig with ethylene, then to 500 psi with air. After two hours reaction time the product mixture was determined by gas chromatography to contain .17g vinyl 2-ethylhexanoate (<1% yield).

**Example 33**

Synthesis of Vinyl Pivalate Pd/Copper(II) Butyrate Catalyst

A palladium (0) colloid containing 10 mL t-butanol and 20 g pivalic acid as the organic medium (palladium (0) concentration .002M), 1.44g copper(II) butyrate (0.2M), and 2.0 g N-methylpyrrolidone (as an internal standard) was charged to a 100 mL Parr reactor. The reaction was heated to 110°C and pressurized to 300 psig with ethylene, then to 500 psi with air. After two hours reaction time the product mixture was determined by gas chromatography to contain .12g vinyl pivalate and .02g acetaldehyde.

**Example 34**

Synthesis of Vinyl Neodecanoate Pd/Copper(II) Neodecanoate Catalyst

A palladium (0) colloid containing 10 mL t-butanol and 20 mL neodecanoic acid as the organic medium (palladium concentration .002M), 2.54g copper(II) neodecanoate (0.2M), and 2.8 g N-methylpyrrolidone (as an internal standard) was charged to a 100 mL Parr reactor. The reaction was heated to 110°C and pressurized to 300 psig with ethylene, then to 500 psig with air. The reaction was vented, sampled, and repressurized every 30 minutes for two hours as in the previous examples. After two hours reaction time the product mixture was determined by gas chromatography to contain .37g vinyl neodecanoate.

**Example 35**

Synthesis of Vinyl Benzoate Pd/Copper(II) Benzoate Catalyst

A palladium (0) colloid containing 20 mL t-butanol and 10g benzoic acid as the organic medium (palladium (0) concentration .002M). 3.72g copper(II) benzoate (0.4 M), and 2.3 g N-methylpyrrolidone (as an internal standard) was charged to a 100 mL Parr reactor. The reaction was heated to 110°C and pressurized to 300 psig with ethylene, then to 500 psi with air. The reaction was vented, sampled, and repressurized every 30 minutes as in the previous examples. After one hour reaction time the product mixture was determined by gas chromatography to contain 1.07g vinyl benzoate and .15g acetaldehyde.

Examples 36 and 37 below illustrate the range of concentrations at which diglyme is effective in the process of the present invention as a promoter at approximately 10-50% by weight of the reaction mixture.

**Example 36**

Synthesis of vinyl 2-ethylhexanoate, Pd/Copper(II) 2-ethylhexanoate/LiCl catalyst 10% diglyme promoter.

A palladium (0) colloid containing 2.7 mL 2-ethylhexanoic acid and 3 mL diglyme as the organic medium (palladium (0) concentration .002M, 10% diglyme), 4.33g copper 2-ethylhexanoate (0.4M), 0.13g lithium chloride (0.2M), and 2.21g N-methylpyrrolidone (as an internal standard) was charged to a 100 mL Parr reactor. The reaction was heated to 110°C and pressurized to 300 psig with ethylene, then to 500 psi with air. The reaction was vented and repressurized every 30 minutes as in the previous examples. After two hours total reaction time the product contained 7.5g vinyl 2-ethylhexanoate, compared with 5.1g vinyl 2-ethylhexanoate obtained without diglyme present (see Example 37 below).

**Example 37**

Synthesis of vinyl 2-ethylhexanoate, Pd/Copper(II) 2-ethylhexanoate/LiCl catalyst 60% diglyme promoter.

A palladium (0) colloid containing 12 mL 2-ethylhexanoic acid, and 18 mL diglyme as the organic medium (palladium (0) concentration .002M, 60% diglyme), 4.32g copper(II) 2-ethylhexanoate (0.4M), 0.13g lithium chloride (0.1M) and 2.06g N-methylpyrrolidone (as an internal standard) was charged to a 100 mL Parr reactor. The reaction was heated to 110°C and pressurized to 300 psig with ethylene, then to 500 psi with air. The reaction was vented and repressurized every 30 minutes as in the previous examples. After two hours total reaction time the product contained 6.6g vinyl 2-ethylhexanoate, compared with 5.1g vinyl 2-ethylhexanoate obtained without diglyme present, and 7.5g obtained with 10% diglyme.

Examples 38, 39 and 40 below (when compared with Examples 3, 22 and 28 above) illustrate the variability of yields that can be obtained in the practice of the present invention.

## Example 38

Synthesis of vinyl 2-ethylhexanoate, Pd/Copper(II) 2-ethylhexanoate semi-continuous air addition.

Thirty mL of a palladium (0) colloid containing 2-ethylhexanoic acid as the organic medium (palladium (0) concentration .002M), 4.21g copper(II) 2-ethylhexanoate (0.4M), and 2.3 g N-methylpyrrolidone (as an internal standard) was charged to a 100 mL Parr reactor. The reaction was heated to 110°C and pressurized to 300 psig with ethylene, then to 500 psi with air. Every 30 minutes, the atmosphere was vented to purge the system of built-up nitrogen, the reactor was freshly recharged with 300 psi ethylene, 200 psi air, and then the mixture was analyzed by gas chromatography. After two hours total reaction time the product mixture contained 1.1g vinyl 2-ethylhexanoate.

## Example 39

Synthesis of vinyl 2-ethylhexanoate, Pd/Copper(II) 2-ethylhexanoate/LiCl.

Thirty mL of a palladium (0) colloid containing 2-ethylhexanoic acid as the organic medium (palladium (0) concentration .002M), 4.23g copper(II) 2-ethylhexanoate (0.4M), 0.12g lithium chloride (0.1M), and 2.31g N-methylpyrrolidone (as an internal standard) was charged to a 100 mL Parr reactor. The reaction was heated to 110°C and pressurized to 300 psig with ethylene, then to 500 psi with air. The reaction was vented and repressurized every 30 minutes as in the previous example. After two hours total reaction time the product mixture contained 5.1g vinyl 2-ethylhexanoate, compared with 1.1g vinyl 2-ethylhexanoate using $Pd/Cu(2EH)_2$ alone.

## Example 40

Synthesis of vinyl pivalate Pd/Copper(II) 2-ethylhexanoate /LiCl.

Thirty 30 mL of a palladium (0) colloid containing 10 mL t-amyl alcohol, 10 mL diglyme and 10g pivalic acid as the organic medium (palladium (0) concentration .002M), 4.31g copper 2-ethylhexanoate (0.4M), 0.13g lithium chloride (0.1M), and 2.35g N-methylpyrrolidone (as an internal standard) was charged to a 100 mL Parr reactor. The reaction was heated to 110°C and pressurized to 300 psig with ethylene, then to 500 psi with air. The reaction was vented and repressurized every 30 minutes as in the previous examples. After two hours total reaction time the product mixture contained 2.9g vinyl pivalate and 1.4g vinyl 2-ethylhexanoate.

Example 41 below illustrates that washing the reaction vessel with lithium chloride as a "preconditioning" step increases the yield of vinyl ester.

## Example 41

Synthesis of vinyl 2-ethylhexanoate Pd/Copper(II) 2-ethylhexanoate LiCl wash.

A 100 mL Parr reactor was charged with 30 mL of a 1 molar solution of lithium chloride dissolved in 2-ethylhexanoic acid. The solution was heated to 110°C and stirred in the reactor for 16 hours after which the reactor was dismantled and subjected to a normal deaning procedure of washing with water, aqua regia, and acetone.

Thirty mL of a palladium (0) colloid containing 2-ethylhexanoic acid as the organic medium (palladium (0)concentration .002M), 4.30g copper(II) 2-ethylhexanoate (0.4M), and 2.3g N-methylpyrrolidone (as an internal standard) was charged to the Parr reactor. The reaction was heated to 110°C and pressurized to 300 psig with ethylene, then to 500 psi with air. Every 30 minutes, the atmosphere was vented and repressurized as in previous examples. After two hours total reaction time the product mixture contained 2.3g vinyl 2-ethylhexanoate, compared with 1.1g obtained when the reactor was not conditioned with LiCl.

Examples 42 to 43 below illustrate that palladium (0) colloid produces a more active catalyst than palladium(II) acetate or palladium(II) acetylacetonate.

## Example 42

Synthesis of vinyl 2-ethylhexanoate Pd acetate/Copper(II) 2-ethylhexanoate.

A catalyst solution consisting of .014g palladium(II) acetate dissolved in 30 mL 2-ethylhexanoic acid (palladium(II) concentration .002M), 4.51g copper(II) 2-ethylhexanoate (0.4M), .04g polyvinylpyrrolidone as a stabilizer, and 2.3g N-methylpyrrolidone (as an internal standard) was charged to a 100 mL Parr reactor. The reaction was heated to 110°C and pressurized to 300 psig with ethylene, then to 500 psi with air. Every 30 minutes, the atmosphere was vented and repressurized as in previous examples. After two hours total reaction time the product mixture contained 0.2g vinyl 2-ethylhexanoate, compared with 1.1g using Pd colloid catalyst.

Example 43

Synthesis of vinyl 2-ethylhexanoate, Pd(II) acetylacetonate/Copper(II) 2-ethylhexanoate.

A catalyst solution consisting of .024g palladium(II) acetylacetonate dissolved in 30 mL 2-ethylhexanoic acid (palladium(II) concentration .002M), 4.52g copper(II) 2-ethylhexanoate (0.4M), .06g polyvinylpyrrolidone as a stabilizer, and 2.1g N-methylpyrrolidone (as an internal standard) was charged to a 100 mL Parr reactor. The reaction was heated to 110°C and pressurized to 300 psig with ethylene, then to 500 psi with air. Every 30 minutes, the atmosphere was vented and repressurized as in previous examples. After two hours total reaction time the product mixture contained 0.5g vinyl 2-ethylhexanoate, compared with 1.1g using Pd colloid catalyst.

## Claims

1. A homogeneous liquid phase process for producing a vinyl carboxylate in which the carboxylate group contains at least three carbon atoms, which process comprises (1) forming a homogeneous liquid reaction mixture containing ethylene, oxygen, carboxylic acid having at least four carbon atoms, a palladium (II) catalyst and a copper (II) oxidant and (2) maintaining the reaction mixture under conditions at which the ethylene and the carboxylic acid react to form the vinyl carboxylate in the homogeneous liquid reaction mixture, with the proviso that at least one of the following conditions exists during said process:
   (i) the copper (II) oxidant is a copper (II) carboxylate in which carboxylate group has a molecular weight that is higher than the molecular weight of the carboxylate group in the carboxylic acid;
   (ii) the palladium (II) catalyst is formed in situ in the reaction mixture by the oxidation of a palladium (0) colloid; and/or
   (iii) the reaction mixture also contains a polyglyme compound as a promoter.

2. A homogeneous liquid phase process for producing a vinyl carboxylate in which the carboxylate group contains at least three carbon atoms as claimed in Claim 1, which process comprises (1) forming a homogeneous liquid reaction mixture containing ethylene, oxygen, carboxylic acid having at least-four carbon atoms, a palladium (II) catalyst and a copper (II)oxidant and (2) maintaining the reaction mixture under conditions at which the ethylene and the carboxylic acid react to form the vinyl carboxylate in the homogeneous liquid reaction mixture, with the proviso that the copper (II) oxidant is a copper (II) carboxylate in which carboxylate group has a molecular weight that is higher than the molecular weight of the carboxylate group in the carboxylic acid.

3. A homogeneous liquid phase process for producing a vinyl carboxylate in which the carboxylate group contains at least three carbon atoms as claimed in Claim 1, which process comprises (1) forming a homogeneous liquid reaction mixture containing ethylene, oxygen, carboxylic acid having at least four carbon atoms, a palladium (II) catalyst and a copper (II) oxidant and (2) maintaining the reaction mixture under conditions at which the ethylene and the carboxylic acid react to form the vinyl carboxylate in the homogeneous liquid reaction mixture, with the proviso that the palladium (II) catalyst is formed in situ in the reaction mixture by the oxidation of a palladium (0) colloid.

4. A homogeneous liquid phase process for producing a vinyl carboxylate in which the carboxylate group contains at least three carbon atoms as claimed in Claim 1, which process comprises (1) forming a homogeneous liquid reaction mixture containing ethylene, oxygen, carboxylic acid having at least four carbon atoms, a palladium (II) catalyst and a copper (II) oxidant and (2) maintaining the reaction mixture under conditions at which the ethylene and the carboxylic acid react to form the vinyl carboxylate in the homogeneous liquid reaction mixture, with the proviso that the reaction mixture also contains a polyglyme compound as a promoter.

5. A process as claimed in claim 1 wherein the carboxylic acid is butyric acid, crotonic acid, pivalic acid, 2-ethylhexanoic acid, neodecanoic acid or benzoic acid.

6. A process as claimed in claim 1 wherein the copper (II) oxidant is copper 2-ethylhexanoate, copper butyrate, copper neodecanoate copper acetate and copper benzoate.

7. A process as claimed in claim 1 wherein lithium chloride is used as a promoter.

8. A process as claimed in claim 1 wherein the carboxylic acid is butyric acid, crotonic acid, pivalic acid, adipic acid or benzoic acid and the copper (II) oxidant is copper 2- ethylhexanoate.

European Patent Office

# EUROPEAN SEARCH REPORT

Application Number
EP 94 30 7138

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | GB-A-1 067 850 (IMPERIAL CHEMICAL INDUSTRIES LTD)<br>* page 3; example 1 *<br>* page 4; claims *<br>--- | 1 | C07C67/055<br>C07C69/01 |
| A | GB-A-1 227 279 (SOCIETA ITALIANA RESINE S.P.A.)<br>* page 2 - page 3; example 1 *<br>* page 4; claims *<br>----- | 1 | |
| | | | TECHNICAL FIELDS SEARCHED (Int.Cl.6) |
| | | | C07C |

The present search report has been drawn up for all claims

| Place of search | Date of completion of the search | Examiner |
|---|---|---|
| THE HAGUE | 5 January 1995 | Kinzinger, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)